# EUROPÄISCHE PATENTSCHRIFT

(11) **EP 2 412 341 B1**
(45) Veröffentlichungstag und Bekanntmachung des Hinweises auf die Patenterteilung: **20.05.2015**
(21) Anmeldenummer: 10007881.5
(22) Anmeldetag: 29.07.2010
(51) Int. Cl.: A61F 9/008, B23K 26/08

(54) **Vorrichtung zum Bearbeiten von Augengewebe mittels Femtosekundenlaserpulsen**
Device for machining eye tissue using femtosecond laser pulses
Dispositif destiné au traitement de tissu oculaire à l'aide d'impulsions laser à femtosecondes

(43) Veröffentlichungstag der Anmeldung: 01.02.2012
(60) Teilanmeldung: 15001012.2
(73) Patentinhaber: SIE AG, Surgical Instrument Engineering, 2562 Port (CH)
(72) Erfinder: Rathjen, Christian, 28197 Bremen (DE)
(74) Vertreter: Rentsch Partner AG

(56) Entgegenhaltungen:
- EP-A1- 0 176 872
- EP-A1- 1 486 185
- EP-A1- 1 897 520
- EP-B1- 1 731 120
- US-A1- 2008 049 285

## Beschreibung

### Technisches Gebiet

Die vorliegende Erfindung betrifft eine ophthalmologische Vorrichtung zum Bearbeiten von Augengewebe mittels Femtosekundenlaserpulsen. Die vorliegende Erfindung betrifft insbesondere eine ophthalmologische Vorrichtung mit einer Projektionsoptik zur fokussierten Projektion der Femtosekundenlaserpulse ins Augengewebe.

### Stand der Technik

Zum Bearbeiten von Augengewebe mittels Laserstrahlen wird ein Bildbereich respektive ein Arbeitsbereich mit Laserpulsen gescannt (abgetastet), indem der gepulste Laserstrahl mittels geeigneter Scannersysteme (Ablenkvorrichtungen) in ein oder zwei Scannrichtungen (Abtastrichtungen) abgelenkt wird. Die Ablenkung der Lichtstrahlen respektive der Laserpulse, beispielsweise Femtosekundenlaserpulse, wird im Allgemeinen mit beweglichen Spiegeln vorgenommen, die um eine oder zwei Scannachsen schwenkbar sind, beispielsweise mit Galvanoscannern, Piezoscannern oder Polygonscannern.

In US 7,621,637 wird eine Vorrichtung zum Bearbeiten von Augengewebe beschrieben, welche eine Basisstation mit einer Laserquelle zur Erzeugung von Laserpulsen und einen in der Basisstation angeordneten Scanner mit beweglichen Ablenkspiegeln zum Ablenken der Laserpulse in einer Scannrichtung aufweist. Die abgelenkten Laserpulse werden über ein optisches Übertragungssystem von der Basisstation zu einem Applikationskopf übertragen, der mittels eines mechanisch bewegten Lichtprojektors ein Arbeitsbereich gemäss einem Scannmuster abfährt. Die im Vergleich zur mechanischen Bewegung viel schnellere Ablenkung in der Scannrichtung wird im Applikationskopf auf die mechanische Bewegung des Lichtprojektors und somit auf dessen Scannmuster überlagert. Ein Fast-Scannersystem in der Basisstation ermöglicht eine feine Bewegung der Laserpulse (Microscann), welche auf das Scannmuster des beweglichen Lichtprojektors überlagert wird, das einen grossen Arbeitsbereich abdeckt, beispielsweise das gesamte Auge.

Eine ähnliche Technik wird in den Dokumenten EP 1 897 520 und EP 1 486 185 offenbart.

Mit der Verfügbarkeit von schnelleren Laserquellen, die zunehmend höhere Pulsraten liefern, beispielsweise über eine Million Pulse pro Sekunde (MHz), stossen die bekannten Scannersysteme an ihre physikalischen Grenzen, Pulse separiert platzieren zu können, und die Pulsrate der Laser muss künstlich reduziert werden. Insbesondere die mechanische Bewegung von Lichtprojektoren oder Linsen zum Scannen von Arbeitsbereichen, und auch die Massenträgheit von Galvanometerscannersystemen, die nicht beliebig hohe Beschleunigungen erlaubt, limitiert die möglichen Scannmuster und Scanntrajektorien in dem Sinne, dass starke Richtungsänderungen vermieden werden müssen, und dass die Pulsrate (aufwändig) aktiv reduziert werden muss oder der Laser bei Unterschreitung einer minimalen Scanngeschwindigkeit ausgeschaltet werden muss, beispielsweise in Umkehrpunkten. Somit setzen die bekannten Scannersysteme signifikante Grenzen an ausführbare Schnittführungen. Aus klinischer Sicht ist es aber gewünscht, den Schnittverlauf nach dem biomechanischen Verhalten des Gewebes zu planen und nicht unbedingt nach der Geschwindigkeit und Bandbreite des Scannersystems, wie es mit den bekannten Scannersystemen erfolgt. Im Unterschied zur Oberflächenbearbeitung kann beim Schneiden in weichem Gewebe, beispielsweise Augengewebe, nicht immer mit einfachen Pulsrastern, beispielsweise Zeilen- oder Spiralmuster, gearbeitet werden, da durch interne Gasentwicklung oder Freiwerden von Spannungen Gewebedeformationen verursacht werden können, die es durch geeignet komplexere Scann- respektive Abtastmuster unter Berücksichtigung des erwarteten biomechanischen Verhaltens des Gewebes zu vermeiden gilt. Die bekannten Scannersysteme ermöglichen zwar die Bearbeitung von einfachen Scannmustern, beispielsweise das Schneiden eines Gewebelappens (Flap), das in der Regel als grosses Flächenstück mit einfacher Randgeometrie ausgeführt wird. Bei vereinzelten Bearbeitungsgebieten mit komplizierter Randgeometrie, wie sie zum Beispiel zur refraktiven Korrektur benötigt werden, oder bei anderen, biomechanisch bedingten komplexeren Scannrespektive Abtastmustern, ist das allerdings nicht mehr so einfach möglich. Beispielsweise muss dann ein grossflächiges Scannmuster mit einer Maske belegt werden (elektronisch oder optisch), oder die kleinen Bereiche werden einzeln bearbeitet, z.B. abgerastert, was eine entsprechende Reduktion der Verarbeitungsgeschwindigkeit zur Folge hat, da das Scannersystem bezogen auf die zu scannende Strecke sehr häufig abbremsen und beschleunigen muss.

### Darstellung der Erfindung

Es ist eine Aufgabe der vorliegenden Erfindung eine ophthalmologische Vorrichtung zum Bearbeiten von Augengewebe mittels Femtosekundenlaserpulsen vorzuschlagen, welche zumindest einige Nachteile der bekannten Systeme nicht aufweist. Es ist insbesondere eine Aufgabe der vorliegenden Erfindung eine ophthalmologische Vorrichtung zum Bearbeiten von Augengewebe mittels Femtosekundenlaserpulsen vorzuschlagen, welche ohne mechanische Bewegung von Linsen für das Scannen eines Bearbeitungsgebiets auskommt und den Einsatz von Laserquellen mit hohen Pulsfrequenzen, insbesondere Pulsfrequenzen im MHz-Bereich mit über einer Million Pulsen pro Sekunde, zur Reduzierung der Bearbeitungszeit und eine flexiblere Schnittführung ermöglicht.

Gemäss der vorliegenden Erfindung werden diese Ziele durch die Merkmale des unabhängigen Anspruchs erreicht. Weitere vorteilhafte Ausführungsformen gehen ausserdem aus den abhängigen Ansprüchen und der Beschreibung hervor.

Die oben genannten Ziele werden durch die vorliegende Erfindung insbesondere dadurch erreicht, dass eine ophthalmologische Vorrichtung zum Bearbeiten von Augengewebe, insbesondere über das gesamte Auge, mittels Femtosekundenlaserpulsen, welche eine Projektionsoptik zur fokussierten Projektion der Femtosekundenlaserpulse ins Augengewebe umfasst, zudem versehen ist mit einem ersten der Projektionsoptik vorgelagerten strahlablenkenden Scannersystem zum Scannen des Augengewebes mit den Femtosekundenlaserpulsen entlang einer Bearbeitungslinie, einem zweiten dem ersten Scannersystem vorgelagerten strahlablenkenden Scannersystem zum Scannen des Augengewebes mit den Femtosekundenlaserpulsen auf einer der Bearbeitungslinie überlagerten Scannkurve, mit einer Scanngeschwindigkeit, die ein Vielfaches der Scanngeschwindigkeit des ersten Scannersystems beträgt, umfassend mindestens ein Ablenkungsspiegel und eine erste Scannachse und eine zweite Scannachse, und einem Steuermodul zum Steuern der Ablenkung der Femtosekundenlaserpulse um die erste Scannachse und der Ablenkung der Femtosekundenlaserpulse um die zweite Scannachse gemäss einer definierten Kurvenform, einer definierten Kurvenamplitude und einer definierten Kurvenausrichtung der Scannkurve, dadurch gekennzeichnet, dass das Steuermodul eingerichtet ist, abhängig von der Richtung der Bearbeitungslinie die Auslenkungsamplitude der Ablenkung um die erste Scannachse und die Auslenkungsamplitude um die zweite Scannachse zu steuern.

Bei spiegelbasierten Scannersystemen ist der Begriff Scannachse als gleichwertig zur Spiegelachse zu verstehen, so dass eine Auslenkung des Spiegels um die Scannachse eine Ablenkung eines Laserstrahls in einer in der Ablenkungsebene verlaufende Scannrichtung bewirkt. Bei anderen Scannersystem, welche keine Spiegelachse aufweisen, ist der Begriff Scannachse als virtuelle Achse zu verstehen, um die ein Spiegel gedreht werden müsste, um den Laserstrahl in die betreffende Scannrichtung abzulenken.

Aus gerätetechnisch praktischen Gründen und aus Kostengründen ist es erforderlich, die Grösse (z.B. der Durchmesser) der Projektions- und Übertragungsoptiken so zu begrenzen, dass in der Regel Auslenkungs- respektive Scannwinkel von deutlich kleiner als 90 Grad sinnvoll realisierbar sind. Bei sehr kleinem Fokus der Laserpulse (Spots), wie sie für einen gewebeschonende und präzise Bearbeitung erforderlich sind, beispielsweise Spots mit einem Durchmesser kleiner als 5µm, insbesondere kleiner als 3µm, vorzugsweise kleiner als 1µm, und grossem Bildfeld (Bearbeitungsbereich) müssen daher grosse Strahldurchmesser gescannt werden, was grosse schwere Spiegel und damit, wie bei Lösungen mit mechanisch bewegten Linsen, geringe Scannfrequenzen bedingt. Mit anderen Worten: Grosse Bildfelder können aus fundamentalen physikalischen Gründen nicht mit kleinen (d.h. schnell ablenkbaren) Spiegeln gescannt werden und gleichzeitig für die Bearbeitungsqualität wünschenswert kleine Spotdurchmesser haben.

Durch das Hintereinanderschalten (Kaskadierung) der zwei strahlablenkenden Scannersystem mit beweglichen Ablenkspiegeln wird eine flexibel konfigurier- und steuerbare Bearbeitung von Augengewebe ermöglicht, wobei das erste Scannersystem ein ausgedehntes Bearbeitungsgebiet abdeckt, beispielsweise das gesamte Auge, und das vorgeschaltete zweite Scannersystem eine schnelle feine Scannbewegung überlagert, deren Form, Grösse und Ausrichtung flexibel einstellbar ist, ohne dass übergrosse Projektions- und Übertragungsoptiken eingesetzt werden müssen, und ohne dass für die Abtastung eine mechanische Bewegung von Linsen oder Projektionsobjektiven erforderlich ist, so dass hohe Scannfrequenzen respektive Abtastraten realisierbar sind. Die Anordnung des vergleichsweise schnelleren zweiten Scannersystem, das "ultraschnelle Scannersystem", zwischen der Laserquelle und dem ersten Scannersystem, das "schnelle Hauptscannersystem", erlaubt die Verwendung kleinerer Strahlaperturen, z.B. Spiegel, und ermöglicht damit ein schnelles, hochfrequentes Scannen des Augengewebes. Dabei kann das schnellere zweite Scannersystem auf hochfrequentes Abtasten und Bearbeiten, d.h. Schneiden, des Augengewebes mit relativ kleiner Auslenkung optimiert werden, und das erste Scannersystem auf das schnelle Anfahren von beliebig adressierbaren Punkten im ausgedehnten Bearbeitungsgebiet (Bildbereich).

In einer Ausführungsvariante ist das Steuermodul eingerichtet, die Kurvenausrichtung der Scannkurve durch gekoppelte Steuerung von der Auslenkungsamplitude der Ablenkung um die erste Spiegelachse und von der Auslenkungsamplitude der Ablenkung um die zweite Spiegelachse zu bestimmen. Durch die einfache Steuerung der Kurvenausrichtung basierend auf den Auslenkungsamplituden wird ein kostspieliger und langsamer mechanischer und/oder optischer Bildrotator hinfällig, da die Steuerung gänzlich über elektronische und/oder programmtechnische Mittel erfolgt.

In einer Ausführungsvariante ist das Steuermodul eingerichtet, die Kurvenausrichtung der Scannkurve durch gekoppelte Steuerung von der Auslenkungsamplitude der Ablenkung um die erste Spiegelachse, von der Auslenkungsamplitude der Ablenkung um die zweite Spiegelachse, und von der relativen Phase von der Ablenkung um die erste Spiegelachse und der Ablenkung um die zweite Spiegelachse zu bestimmen. Durch die gekoppelte Steuerung der Auslenkungsamplituden und relativen Phase kann beispielsweise die Ausrichtung einer Ellipse gesteuert werden.

In einer weiteren Ausführungsvariante ist das Steuermodul eingerichtet, die Kurvenamplitude der Scannkurve durch gekoppelte Steuerung von der Auslenkungsamplitude der Ablenkung um die erste Spiegelachse und von der Auslenkungsamplitude der Ablenkung um die zweite Spiegelachse zu bestimmen.

In einer Ausführungsvariante ist das Steuermodul eingerichtet, die Kurvenform der Scannkurve durch Steuerung der relativen Phase zwischen der Ablenkung um die erste Spiegelachse und der Ablenkung um die zweite Spiegelachse zu bestimmen.

In einer weiteren Ausführungsvariante ist das Steuermodul eingerichtet, die Kurvenform der Scannkurve durch gekoppelte Steuerung von der Auslenkungsamplitude der Ablenkung um die erste Spiegelachse und von der Auslenkungsamplitude der Ablenkung um die zweite Spiegelachse zu bestimmen.

In einer weiteren Ausführungsvariante ist das Steuermodul eingerichtet, die Kurvenform der Scannkurve durch gekoppelte Steuerung der Ablenkungsfrequenz um die erste Spiegelachse und der Ablenkungsfrequenz um die zweite Spiegelachse zu bestimmen.

In einer Ausführungsvariante ist das Steuermodul eingerichtet, eine durch Fouriersynthese definierbare Kurvenform der Scannkurve durch Steuerung der Auslenkungsamplitude der Ablenkung um die erste Spiegelachse, der Auslenkungsamplitude der Ablenkung um die zweite Spiegelachse, der relativen Phase zwischen der Ablenkung um die erste Spiegelachse und der Ablenkung um die zweite Spiegelachse, der Ablenkungsfrequenz um die erste Spiegelachse, und/oder der Ablenkungsfrequenz um die zweite Spiegelachse.

Vorzugsweise ist das Steuermodul eingerichtet, das erste Scannersystem und das zweite Scannersystem gekoppelt zu steuern.

Vorzugsweise ist das Steuermodul eingerichtet, abhängig von der Bearbeitungslinie die Auslenkungsamplitude der Ablenkung um die erste Spiegelachse, die Auslenkungsamplitude der Ablenkung um die zweite Spiegelachse, die relative Phase zwischen der Ablenkung um die erste Spiegelachse und der Ablenkung um die zweite Spiegelachse, die Ablenkungsfrequenz um die erste Spiegelachse und/oder die Ablenkungsfrequenz um die zweite Spiegelachse zu steuern.

In einer Ausführungsvariante ist das Steuermodul eingerichtet, bei einer Richtungsänderung der Bearbeitungslinie die Auslenkungsamplitude der Ablenkung um die erste Spiegelachse zu erhöhen und die Auslenkungsamplitude der Ablenkung um die zweite Spiegelachse zu reduzieren. Die Reduktion respektive Erhöhung der Auslenkungsamplituden erfolgt beispielsweise kontinuierlich.

In einer weiteren Ausführungsvariante ist das Steuermodul eingerichtet, die Ablenkung um die erste Spiegelachse und die Ablenkung um die zweite Spiegelachse so zu steuern, dass die Ablenkung um die erste Spiegelachse mit einer doppelt so hohen Ablenkungsfrequenz wie die Ablenkung um die zweite Spiegelachse erfolgt und die Ablenkung um die erste Spiegelachse eine mindestens teilweise Kompensation der durch das erste Scannersystem bewirkten Ablenkung in Richtung der Bearbeitungslinie bewirkt.

In einer weiteren Ausführungsvariante umfasst die Vorrichtung eine zwischen dem ersten Scannersystem und dem zweiten Scannersystem angeordnete Blende zum Ausblenden von Femtosekundenlaserpulsen, die vom zweiten Scannersystem in einen Bereich ausserhalb einer definierten Scannbreite abgelenkt werden.

In einer weiteren Ausführungsvariante umfasst die Vorrichtung ein steuerbares Filtermodul zum selektiven Ausschliessen von Femtosekundenlaserpulsen in einem definierten Bereich der Scannkurve.

Vorzugsweise sind die erste Spiegelachse und die zweite Spiegelachse des zweiten Scannersystems orthogonal zueinander ausgerichtet sind, das zweite Scannersystem ist eingerichtet, die Femtosekundenlaserpulse oszillierend um die erste Spiegelachse und um die zweite Spiegelachse abzulenken, und das erste Scannersystem weist einen im Vergleich zum zweiten Scannersystem wesentlich grösseren Auslenkungsgrad auf.

In einer Ausführungsvariante ist das Steuermodul eingerichtet, die Ablenkung der Femtosekundenlaserpulse um die erste Spiegelachse und die Ablenkung der Femtosekundenlaserpulse um die zweite Spiegelachse so zu steuern, dass die Scannkurve eine runde Kurvenform aufweist, und das Steuermodul ist eingerichtet, die Scanngeschwindigkeit des ersten Scannersystems so zu steuern, dass sich aus der Überlagerung von der Scannkurve auf die Bearbeitungslinie eine resultierende Scanntrajektorie mit einem definierten Trajektorienverlauf ergibt. Bei der Überlagerung einer kreisrunden Scannkurve auf die Bearbeitungslinie wird aufgrund der Symmetrie eine Steuerung der Scannkurve hinsichtlich ihrer Ausrichtung entbehrlich.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
- Figur 1:: zeigt ein Blockdiagramm, welches schematisch eine ophthalmologische Vorrichtung mit zwei hintereinandergeschalteten Scannersystemen zum Bearbeiten von Augengewebe mittels Femtosekundenlaserpulsen illustriert.
- Figur 1a:: zeigt schematisch eine Projektionsebene eines der Scannersysteme mit zwei orthogonal zueinander ausgerichteten Scannachsen.
- Figur 1b:: zeigt schematisch eine Bearbeitungsebene mit einer aus der Hintereinanderschaltung der Scannersysteme resultierenden Scanntrajektorie.
- Figur 2:: illustriert die Überlagerung von nicht phasenverschoben schwingenden Scannbewegungen um zwei orthogonal zueinander angeordnete Spiegel- oder Scannachsen.
- Figur 3:: illustriert die Überlagerung von um π/2 phasenverschoben schwingenden Scannbewegungen um zwei orthogonal zueinander angeordnete Spiegel- oder Scannachsen.
- Figur 4:: illustriert die Überlagerung von um π/2 phasenverschoben und mit unterschiedlichen Frequenzen schwingenden Scannbewegungen um zwei orthogonal zueinander angeordnete Spiegel- oder Scannachsen.
- Figur 5:: illustriert die Überlagerung einer Scannkurve eines Scannersystems mit zwei Spiegel- oder Scannachsen auf die Bearbeitungslinie eines weiteren Scannersystems, wobei für eine Flankenbegradigung die Ablenkung um eine der Spiegelachsen mit einer doppelt so hohen Ablenkungsfrequenz wie die Ablenkung um die andere Spiegelachse erfolgt.
- Figur 6:: illustriert die Überlagerung einer Scannkurve eines Scannersystems mit zwei Spiegel- oder Scannachsen auf die Bearbeitungslinie eines weiteren Scannersystems, wobei zur Erzeugung eines begradigten Scannmusters jeweils die aufsteigenden Flanken der Scannkurve ausgeblendet werden.
- Figur 7:: illustriert die Überlagerung einer Scannkurve eines Scannersystems mit zwei Spiegel- oder Scannachsen auf die Bearbeitungslinie eines weiteren Scannersystems, wobei zur Einhaltung der Scannbreite und der Ausrichtung zur Bearbeitungslinie bei einer Richtungsänderung der Bearbeitungslinie die Auslenkungsamplituden der Ablenkungen um die Spiegel- oder Scannachsen angepasst werden.
- Figur 8a:: illustriert die Überlagerung einer runden Scannkurve eines Scannersystems mit zwei Spiegel- oder Scannachsen auf eine runde Bearbeitungslinie eines weiteren Scannersystems.
- Figur 8b:: illustriert die Überlagerung der runden Scannkurve auf die runde Bearbeitungslinie, wobei die Scannkurve hinsichtlich der Scannkurve von Figur 8a einen umgekehrten Drehsinn aufweist.

### Wege zur Ausführung der Erfindung

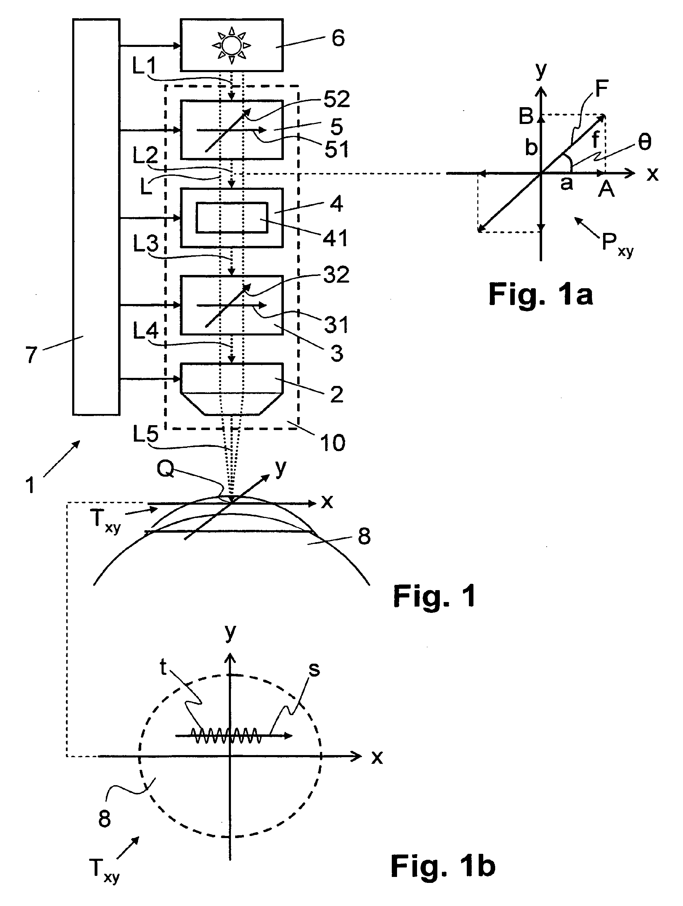

In der Figur 1 bezieht sich das Bezugszeichen 1 auf eine ophthalmologische Vorrichtung zum Bearbeiten von Augengewebe 8 mittels eines gepulsten Laserstrahls L1 mit Femtosekundenlaserpulsen. Der gepulste Laserstrahl L1, vorzugsweise mit Pulsfrequenzen im MHz-Bereich mit über einer Million Pulsen pro Sekunde, wird von einer Strahlquelle 6 geliefert und über ein optisches Übertragungssystem 10 fokussiert entlang einer Scanntrajektorie t als gepulster Bearbeitungsstrahl L5 auf respektive in das Augengewebe 8 projiziert. Die Strahlquelle 6 ist je nach Ausführung Bestandteil des optischen Übertragungssystems 10 oder als separate Einheit ausgestaltet, welche über ein Lichtübertragungssystem, beispielsweise eine Lichtfaserleitung und/oder ein Spiegel-/Linsensystem, mit dem optischen Übertragungssystem 10 verbunden ist.

Wie in der Figur 1 schematisch dargestellt ist, umfasst die ophthalmologische Vorrichtung 1 respektive das optische Übertragungssystem 10 zwei strahlablenkende Scannersysteme 3, 5 und ein optionales Filtermodul 4, die im Strahlengang (L1-L2-L3-L4) von der Strahlquelle 6 zur Projektionsoptik 2 angeordnet sind.

Das strahlablenkende Scannersystem 5 weist eine Scanngeschwindigkeit auf, die ein Vielfaches der Scanngeschwindigkeit des Scannersystems 3 beträgt. Entsprechend werden in der nachfolgenden Beschreibung das Scannersystem 5 als Fast-Scannersystem 5 und das Scannersystem 3 als Slow-Scannersystem 3 bezeichnet. Das Fast-Scannersystem 5 weist somit eine um ein Vielfaches schnellere Ablenkgeschwindigkeit und, bei oszillierenden Scannersystemen, um ein Vielfaches höhere Oszillationsfrequenzen (wₓ, w_{y}) auf, als mit dem Slow-Scannersystem 3 durchführbar wären. Insbesondere weist das Fast-Scannersystem 5 eine höhere Scanngeschwindigkeit im Bildfeld des Projektionsobjektivs (Schneidobjektiv) auf, also im Bildfeld der Projektionsoptik 2, und/oder hat eine höhere Scannfrequenz. Andererseits weist das Slow-Scannersystem 3 einen im Vergleich zum Fast-Scannersystem 5 wesentlich grösseren Auslenkungsgrad im Bildfeld der Projektionsoptik auf. Das Slow-Scannersystem 3 ermöglicht somit ein im Vergleich zum Fast-Scannersystem 5 viel grösseres Bildfeld und Bearbeitungsgebiet abzudecken und anzusteuern, so dass das Augengewebe 8 über das gesamte Auge vollständig gescannt und mit Femtosekundenlaserpulsen bearbeitet werden kann.

Das Fast-Scannersystem 5 ist vorzugsweise zwischen der Strahlquelle 6 und dem Slow-Scannersystem 3 angeordnet. Das Fast-Scannersystem 5 ist beispielsweise im Strahlengang unmittelbar nach der Strahlquelle 6 angeordnet.

Das Slow-Scannersystem 3 ist vorzugsweise zwischen dem Fast-Scannersystem 5 und der Projektionsoptik 2 angeordnet. Das Slow-Scannersystem 3 ist beispielsweise im Strahlengang unmittelbar vor der Projektionsoptik 2 angeordnet.

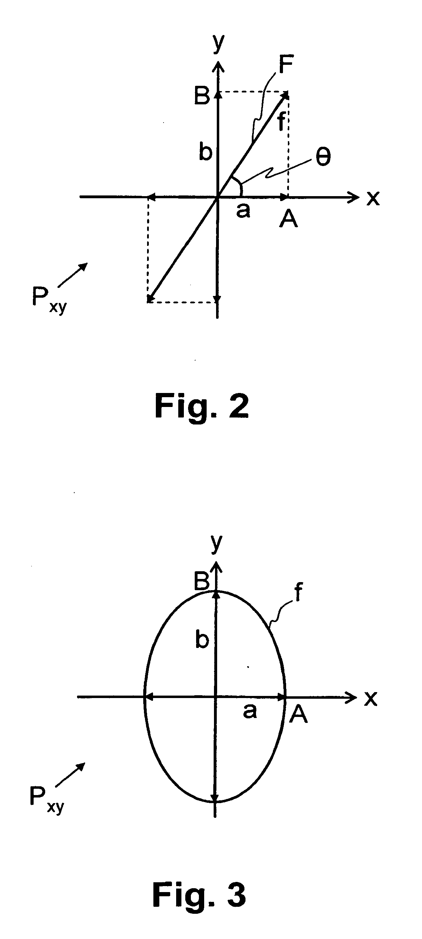

Das Fast-Scannersystem 5 weist zwei Spiegelachsen 51, 52 auf, die vorzugsweise orthogonal zueinander ausgerichtet sind. Die Scannachsen (Spiegelachsen) 51, 52 sind je nach Ausführungsvariante mit einem gemeinsamen, verkippbaren (Tip-Tilt-Modus) Ablenkungsspiegel oder mit jeweils einem eigenen, separaten Ablenkungsspiegel gekoppelt, welche hintereinandergeschaltet angeordnet sind. Die Verwendung eines Scannersystems mit zwei Scannachsen 51, 52 und einem gemeinsamen verkippbaren Ablenkungsspiegel hat den Vorteil, dass sich kostspielige Zwischenoptiken einspaaren lassen und der gesamte Aufbau der Vorrichtung 1 kompakter ausfällt. Das Fast-Scannersystem 5 lenkt den gepulsten Laserstrahl L1 von der Strahlquelle 6 respektive dessen Femtosekundenlaserpulse mit einer definierten Scannbewegung auf eine, in einer Projektionsebene P_{xy} definierte, Scannkurve f ab, wie später mit Bezug zu den Figuren 2, 3 und 4 detaillierter beschrieben wird. Fig. 1a illustriert schematisch eine Projektionsebene P_{xy} des Fast-Scannersystems 5, wobei die Scannachse 51 entlang der x-Achse und die Scannachse 52 entlang der y-Achse ausgerichtet sind. Der Bewegungsvektor a stellt die Auslenkung des Ablenkungsspiegels um die Scannachse 52 (y-Achse) mit einer Auslenkungsamplitude A in x-Richtung, und der Bewegungsvektor b stellt die Auslenkung des Ablenkungsspiegels um die Scannachse 51 (x-Achse) mit einer Auslenkungsamplitude B in y-Richtung dar. Bei einer synchronisierten, d.h. gleichfrequentig (wₓ=w_{y}) und nicht-phasenverschobenen (ϕ=0), Auslenkung um die beiden Scannachsen 51, 52 werden die Femtosekundenlaserpulse entlang einer Scannkurve f abgelenkt, die in Figur 1a durch einen entsprechenden Bewegungsvektor dargestellt ist. Die Auslenkungsamplituden A, B bestimmen somit die Ausrichtung θ und Auslenkungsamplitude F der resultierenden Scannbewegung respektive Scannkurve f des Scannersystems 5 auf der Projektionsebene P_{xy}.

Das Slow-Scannersystem 3 weist vorzugsweise ebenfalls zwei orthogonal zueinander ausgerichtete Scannachsen (Spiegelachsen) auf, die mit einem gemeinsamen, verkippbaren Ablenkspiegel oder mit zwei separaten Ablenkspiegeln gekoppelt sind.

Vorzugsweise ist das Slow-Scannersystem 3 frei adressierbar in Form von zwei Galvanometer-Scannern oder mit einem im Tip-Tilt-Modus verkippbaren zweiachsigen Ablenkungsspiegel Spiegel ausgeführt (z.B. über Piezoelemente angesteuert). Je nach Betriebsmodus oder Konstruktion ist das Fast-Scannersystem 5 als resonanter, oszillierender, oder frei adressierbarer Scanner ausgeführt. Ein sinusförmiger Betriebsmodus erlaubt insbesondere bei mechanischen Resonanzscannern mit Schwingspiegeln (auch als MEM (Micro Electro-Mechanical) Scanner oder mit Piezo-Antrieb) höhere Frequenzen und Ablenkgeschwindigkeiten als mit Galvanometer-Scannern möglich sind. Deshalb werden bevorzugt oszillierende Fast-Scannersysteme 5 im resonanten Betriebsmodus eingesetzt, da diese auf Grund ihrer hohen Scannfrequenzen für den praktischen Einsatz besonders vorteilhaft sind. Weitere Scannertypen, die zum Teil noch höhere Frequenzen erlauben, sind aus der Literatur bekannt (z.B. AOM (Acusto-optische Modulatoren) Scanner oder EOM (Electro-optische Modulatoren)). Vorzugsweise werden das Fast-Scannersystem 5 und das Slow-Scannersystem 3 mit orthogonalen Scannachsen (Spiegelachsen) ausgeführt und betrieben.

Das optionale Filtermodul 4 ist im Strahlengang vorzugsweise zwischen dem Fast-Scannersystem 5 und dem Slow-Scannersystem 3 angeordnet. Je nach Ausführungsvariante umfasst das Filtermodul 4 eine feste und/oder steuerbare Blende 41, die auch als Shutter ausgeführt sein kann, wobei der Shutter vorzugsweise beim Laser angeordnet und unmittelbar der Strahlquelle 6 nachgeschaltet ist. In einer Ausführungsvariante ist die Blende 41 als Feldblende ausgeführt, also in einer Zwischenbildebene angeordnet, wobei überdies je nach Ausführungsvariante die Feldblende variabel und/oder asymmetrisch ausgestaltet ist.

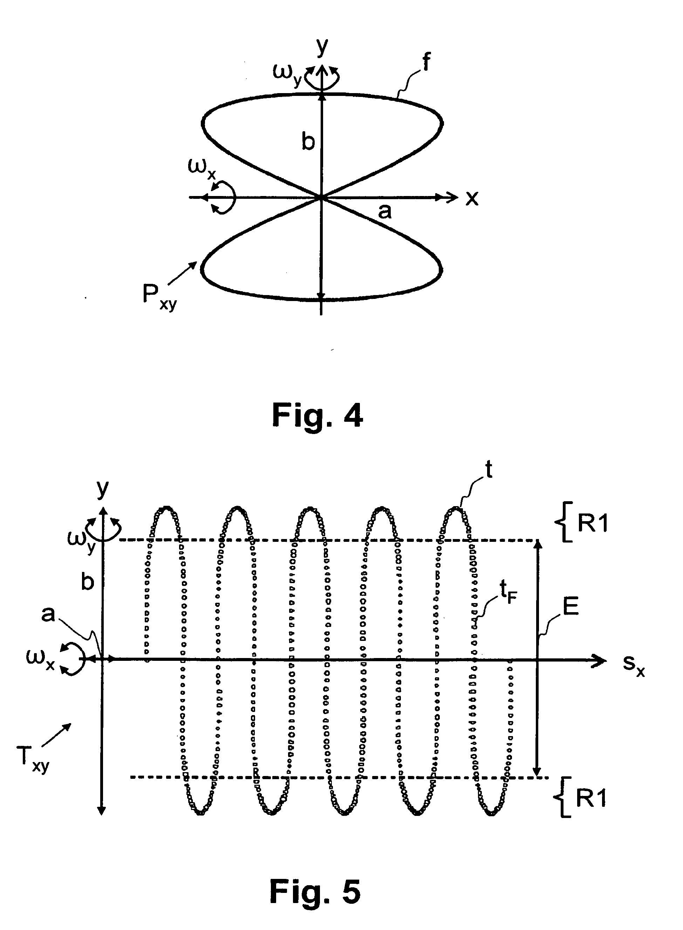

Das Slow-Scannersystem 3 ist eingerichtet das Augengewebe 8 mit den Femtosekundenlaserpulsen in einem ausgedehnten Bearbeitungsbereich entlang einer Bearbeitungslinie s zu scannen. Dabei lenkt das Slow-Scannersystem 3 die vom Fast-Scannersystem 5 abgelenkten Femtosekundenlaserpulse L2 respektive die vom Filtermodul 4 gefilterten und nicht ausgeblendeten Femtosekundenlaserpulse L3 ab. Die Richtung und Form der Bearbeitungslinie s wird wie obenstehend mit Bezug zu Figur 1a für das Fast-Scannersystem 5 beschrieben durch die Auslenkungsamplituden der Auslenkungen um die Scannachsen (Spiegelachsen) 31, 32 des Slow-Scannersystems 3 bestimmt. Die durch das Fast-Scannersystem 5 erzeugte Scannbewegung respektive Scannkurve f wird somit der durch das Slow-Scannersystem 3 kontinuierlich gescannten Bearbeitungslinie s überlagert, wodurch in der Bearbeitungsebene T_{xy}, wie in Figur 1b dargestellt, eine resultierende Scanntrajektorie t gebildet wird, mit der das Augengewebe 8 tatsächlich bearbeitet wird. In den Figuren 5, 6, 7, 8a und 8b sind Beispiele verschiedener erzeugter Scanntrajektorien t zur Bearbeitung des Augengewebes 8 auf einer Bearbeitungsebene T_{xy} dargestellt, auf die später detaillierter eingegangen wird.

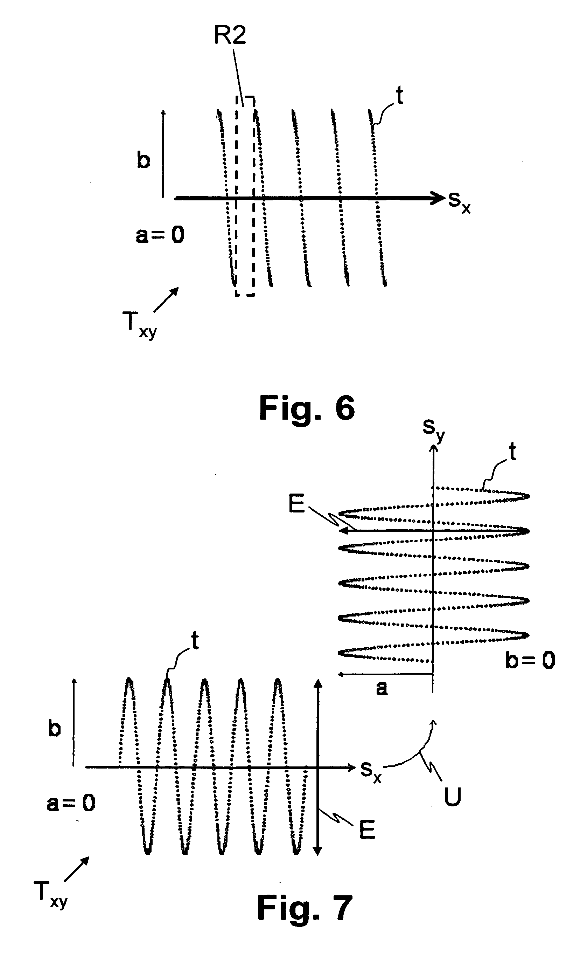

Das Filtermodul 4 ist eingerichtet, gewisse der vom Fast-Scannersystem 5 abgelenkten Femtosekundenlaserpulse L2 gemäss definierten Filterkriterien auszublenden. Die Blende 41 ist beispielsweise fest oder steuerbar eingerichtet, Femtosekundenlaserpulse L2, die vom Fast-Scannersystem 5 in einen Bereich ausserhalb einer definierten Scannbreite E abgelenkt werden auszublenden, oder abgelenkte Femtosekundenlaserpulse L2 vollständig oder in einem definierten Bereich R1, R2 der Scannkurve f auszuschliessen. Im Beispiel der Figur 5, ist das Filtermodul 4 eingerichtet, die Spitzen der Auslenkungen b, beispielsweise über einem definierten Amplitudenwert B, im Bereich R1 auszublenden. Im Beispiel der Figur 6, ist das Filtermodul 4 eingerichtet, jeweils die aufsteigenden Flanken der Scanntrajekorie t im Bereich R2 auszublenden, so dass ein Scannmuster mit nahezu parallelen Scannstrecken erzeugt wird. Die Ausblendbereiche R1, R2 sind beispielsweise dynamisch und frei definier- und veränderbar, beispielsweise abhängig von den Auslenkungen a, b um die Spiegelachsen 51, 52 und/oder von der Richtung der Bearbeitungslinie s.

Die Projektionsoptik 2 ist eingerichtet, die vom Slow-Scannersystem 3 abgelenkten Femtosekundenlaserpulse L4 fokussiert auf respektive in das Augengewebe 8 zu projizieren, wobei das Augengewebe 8 im Fokuspunkt Q, abhängig von der Scanngeschwindigkeit in Richtung der Bearbeitungslinie s, jeweils durch einen einzelnen Femtosekundenlaserpuls oder durch mehrere nacheinander überlappend projizierte Femtosekundenlaserpulse aufgelöst wird. In einer Ausführungsvariante ist die Projektionsoptik 2 zudem eingerichtet den Fokus Q des fokussierten, abgelenkten, gepulsten Laserstrahls L5, beispielsweise durch vertikale Verschiebungen, in der Projektionsrichtung, einzustellen. Ansonsten ist die Projektionsoptik 2 während der Behandlung stationär, d.h. für die Abtastung und Bearbeitung des Augengewebes 8 bedarf es keiner lateralen mechanischen Bewegung (in x- und/oder y-Richtung) von Linsen der Projektionsoptik 2, nachdem diese für die geplante Behandlung auf das Auge des Patienten ausgerichtet wurde. Eine Fixierung am Auge erfolgt beispielsweise mittels eines vakuumgesteuerten Saugrings.

Wie in der Figur 1 ersichtlich ist, umfasst die ophthalmologische Vorrichtung 1 ein Steuermodul 7, das je nach Ausführung als Bestandteil des optischen Übertragungssystems 10 oder als separate Einheit ausgestaltet ist, welche über Steuerleitungen respektive eine oder mehrere Datenkommunikationsverbindungen, beispielsweise mehrere Signal- und/oder Datenleitungen und/oder ein Datenbus, zur Steuerung mit dem optischen Übertragungssystem 10 verbunden ist. Das Steuermodul 7 ist je nach Ausführung mit der Strahlquelle 6, dem Fast-Scannersystem 5, dem Filtermodul 4, dem Slow-Scannersystem 3 und/oder der Projektionsoptik 2 verbunden. Das Steuermodul 7 umfasst vorzugsweise einen oder mehrere Prozessoren und einen greifbaren computerlesbaren Datenträger (Computerprogrammprodukt), welcher fest oder entfernbar mit den Prozessoren verbunden ist und auf welchem mindestens ein programmiertes Softwaremodul gespeichert ist, das Computerprogrammcode zur Steuerung der Prozessoren umfasst. Der Fachmann wird verstehen, dass das Steuermodul 7 in verschiedenen Ausführungsvarianten vollständig oder wenigstens teilweise mit Hardwarekomponenten implementierbar ist.

In den folgenden Abschnitten wird die Funktionalität des Steuermoduls 7 und die dadurch bewirkte Steuerung der Prozessoren und damit der ophthalmologischen Vorrichtung 1 mit Bezug zu den Figuren 2, 3, 4, 5, 6, 7, 8a und 8b beschrieben.

Je nach Ausführungsvariante ist das Steuermodul 7 eingerichtet, die Strahlquelle 6 und/oder die Projektionsoptik 2 zu steuern, beispielsweise hinsichtlich Pulsenergie, Pulsfrequenz respektive Fokustiefe, was jedoch in den nachfolgenden Abschnitten nicht detaillierter beschrieben werden soll.

Das Steuermodul 7 umfasst insbesondere eine Fastscannersteuerung zum Steuern der Ablenkung a, b der Femtosekundenlaserpulse um die Spiegelachsen 51, 52 des Fast-Scannersystems 5. Wie in der Tabelle 1 angegeben ist, ist die Fastscannersteuerung eingerichtet, die Ablenkung a, b um die Spiegelachsen 51, 52 zur Ausführung definierter Scannkurven f zu steuern, welche durch unterschiedliche Kurvenform, Kurvenausrichtung und Kurvenamplitude (respektive Kurvengrösse) definiert sind und beispielsweise eine zugeordnete eindeutige Kurvenkennung aufweisen. Um die Femtosekundenlaserpulse auf eine selektierte definierte Scannkurve f abzulenken verwendet das Steuermodul 7 entsprechende Steuerparameter zur Steuerung des Fast-Scannersystems 5, die der betreffenden Scannkurve f zugeordnet sind. Die Auslenkungen um die Spiegelachsen 51, 52 werden insbesondere mit Parametern zur Steuerung der jeweiligen Auslenkungsamplituden A, B, Auslenkungsfrequenz wₓ, w_{y} und/oder relative Phase ϕ (Phasenverschiebung) zwischen den Auslenkungen a, b (Oszillation) um die Spiegelachsen 51, 52 bestimmt.

**Tabelle 1**

| Scannkurve | | | | Steuerparameter | | | | |
|---|---|---|---|---|---|---|---|---|
| | | | | Auslenkung um erste Scannachse | | Auslenkung um zweite Scannachse | | Phasenverschiebung |
| Kennung | Form | Ausrichtung | Amplitude | Amplitude | Frequenz | Amplitude | Frequenz | |
| ... | ... | ... | ... | ... | ... | ... | ... | ... |

Figur 2 illustriert ein Beispiel wie durch Steuerung der Auslenkungsamplituden A, B um die Scannachsen 51, 52 die Ausrichtung θ und Auslenkungsamplitude F der resultierenden Scannbewegung f auf der Projektionsebene P_{xy} durch das Steuermodul 7 einstellbar sind, wenn die verbleibenden Steuerparameter so gesetzt werden, dass die Ablenkung a, b um die beiden Scannachsen 51, 52 mit gleicher Frequenz ωₓ=ω_{y} und ohne Phasenverschiebung ϕ=0 zwischen den Auslenkungsschwingungen erfolgt.

Figur 3 zeigt ein Beispiel, in welchem zudem mittels eines Phasenstellglieds, beispielsweise ein Teil des Steuermoduls 7, die relative Phase ϕ zwischen den sinusförmig oszillierenden Auslenkungen a, b um die Scannachsen 51, 52 einstellbar ist. Das Beispiel zeigt eine Ansteuerung des Fast-Scannersystems 5 mit einer Phasenverschiebung von ϕ=π/2 zwischen den Auslenkungen um die Scannachsen 51, 52, bei gleichen Auslenkungsamplituden A, B wie im Beispiel der Figur 2, so dass durch das Fast-Scannersystem 5 eine resultierende Scannkurve f mit einer elliptischen Form auf der Projektionsebene P_{xy} erzeugt wird. Die Orientierung respektive Ausrichtung θ der sich ergebenden Ellipse kann durch gleichzeitiges Einstellen der Auslenkungsamplituden A, B und der relativen Phase ϕ eingestellt werden. Bei gleicher Grösse der Auslenkungsamplituden A, B resultiert eine kreisrunde Scannkurve f.

Figur 4 zeigt ein Beispiel wie sich die Form der resultierenden Scannkurve f durch Einstellung und Steuerung der Auslenkungsfrequenzen ωₓ, ω_{y} der Auslenkungen a, b um die Scannachsen 51, 52 weiter durch das Steuermodul 7 bestimmen lässt. Das Beispiel zeigt die Erzeugung einer Lissajous-förmigen resultierenden Scannkurve f bei einer Einstellung der Auslenkungsfrequenz ω_{y} der Auslenkung x in x-Richtung um die Scannachse 52 (y-Achse) durch das Steuermodul 7 auf den doppelten Wert der Auslenkungsfrequenz ωₓ der Auslenkung b in y-Richtung um die Scannachse 51 (x-Achse), ω_{y}-2ωₓ.

Das Steuermodul 7 respektive die Fastscannersteuerung ist eingerichtet, die Ablenkung a, b der Femtosekundenlaserpulse um die Spiegelachsen 51, 52 hinsichtlich ihrer Auslenkungsamplituden A, B, Auslenkungsfrequenzen ωₓ, ω_{y} und relativer Phase ϕ so zu steuern, dass neben klassischen Lissajous-Figuren resultierende Scannkurven f mit sinus-, sägezähn-, trapez- oder rechteckförmigen Schwingungen oder anderen Formen entstehen, die sich über Fouriersynthese herstellen lassen. Bei frei ansteuerbaren Scannachsen 51, 52 können zusätzlich auch nicht periodische Figuren erzeugt werden.

Das Steuermodul 7 umfasst zudem eine Slowscannersteuerung zum Steuern der Ablenkung der Femtosekundenlaserpulse um die Spiegelachsen 31, 32 des Slow-Scannersystems 3. Die Slowscannersteuerung ist beispielsweise eingerichtet, die Ablenkung um die Spiegelachsen 31, 32 gemäss definierten Bearbeitungslinien s zu steuern, welche durch unterschiedliche Bearbeitungsmuster definiert sind und beispielsweise eine zugeordnete eindeutige Musterrespektive Linienkennung aufweisen. Um die Femtosekundenlaserpulse auf eine Bearbeitungslinie s abzulenken, die beispielsweise durch ein gespeichertes Bearbeitungsmuster oder eine Zeitfunktion definiert ist, verwendet das Steuermodul 7 entsprechende Steuerparameter zur Steuerung des Slow-Scannersystems 3, die der betreffenden Bearbeitungslinie s zugeordnet sind.

Bei der Steuerung des Fast-Scannersystems 5 und des Slow-Scannersystem 3 durch das Steuermodul 7 sind im Wesentlichen drei Betriebsmodi unterscheidbar:
a) Im vollständig gekoppelten Betrieb werden sowohl die Auslenkungsamplituden A, B als auch die relative Phase ϕ der Ablenkungen a, b um beiden Scannachsen 51, 52 des Fast-Scannersystems 5 mit dem Slow-Scannersystem 3 synchronisiert (beispielsweise mit einem Startpunkt in T_{xy} oder einem absoluten Phasenwert als Bezugspunkt). Dieser Betriebsmodus respektive die Ausführungsvariante wird dann gewählt, wenn sowohl die Orientierung respektive Ausrichtung θ als auch die Form der Scannkurve f bezüglich und abhängig von der Bearbeitungslinie s gesteuert werden soll, insbesondere abhängig von der Richtung und der Scanngeschwindigkeit (Vorschubgeschwindigkeit) der Bearbeitungslinie s, beispielsweise zur Steuerung der Phasenlage für die bestimmte und definierte (vorsehbare) Platzierung nacheinander folgender Laserpulse auf der resultierenden Scanntrajektorie t.
b) Im teilweise gekoppelten Betrieb wird eine Teilmenge von Auslenkungsamplituden A, B, Auslenkungsfrequenzen ωₓ, ω_{y} und relativer Phase ϕ der beiden Scannachsen 51, 52 des Fast-Scannersystems 5 abhängig von der Bearbeitungslinie s des Slow-Scannersystems 3 gesteuert respektive eingestellt. Dieser Betriebsmodus respektive die Ausführungsvariante wird dann gewählt, wenn vorallem die Orientierung respektive Ausrichtung θ und/oder die Grösse der Scannkurve f bezüglich der Bearbeitungslinie s zum Erzeugen einer gewünschten Scanntrajektorie t gesteuert werden soll.
c) Im entkoppelten Betrieb hat die Orientierung respektive Ausrichtung θ der Scannkurve f bezüglich der Bearbeitungslinie s keine Bedeutung und die Grösse (Amplitude F) ist fest vorgegeben.

Figur 5 zeigt ein Beispiel wie eine Scanntrajektorie t mit begradigten Flanken t_{F} der zur Bearbeitungslinie sₓ orthogonalen Schwingungen der Auslenkung b um die Scannachse 51 erzeugt wird. Wie in der Figur 5 schematisch dargestellt ist, ist das Steuermodul 7 respektive ein Begradigungsmodul eingerichtet, das Fast-Scannersystem 5 derart zu steuern, dass die Auslenkung a in x-Richtung um die Scannachse 52 mit einer doppelten Auslenkungsfrequenz w_{y}-2wₓ der Auslenkung b in y-Richtung um die Scannachse 51 erfolgt. Dabei wird die Auslenkungsamplitude A in Abhängigkeit der Auslenkungsamplitude B und der Scanngeschwindigkeit des Slow-Scannersystems 3 in Richtung der Bearbeitungslinie sₓ so gesetzt, dass die Bewegungskomponenten in der (x-)Richtung der Bearbeitungslinie sₓ bei den auf- und absteigenden Flanken t_{F} der orthogonalen Auslenkungsschwingungen zur Begradigung der Schwingungsflanken t_{F} kompensiert werden (auf das Ausblenden von Teilen der Trajektorie t kann somit vorteilhaft verzichtet werden).

Figur 7 zeigt ein Beispiel wie eine Scanntrajektorie t an eine Richtungsänderung der Bearbeitungslinie sₓ, s_{y}, beispielsweise von der x-Richtung auf die y-Richtung, angepasst wird. Wie in der Figur 7 schematisch dargestellt ist, ist das Steuermodul 7 respektive ein Rotatormodul eingerichtet, das Fast-Scannersystem 5 derart zu steuern, dass es die Auslenkungsamplitude B der Auslenkung b in y-Richtung um die Scannachse 51 und die Auslenkungsamplitude A der Auslenkung a in x-Richtung um die Scannachse 52 abhängig von der Richtung der Bearbeitungslinie sₓ, s_{y} setzt, beispielsweise so, dass bei der Bearbeitung durch das Slow-Scannersystem 3 entlang der Bearbeitungslinie sₓ in x-Richtung die Auslenkungsamplitude B des Fast-Scannersystems 5 auf eine definierte Scannbreite E und die Auslenkungsamplitude A des Fast-Scannersystems 5 auf Null gesetzt wird, und umgekehrt bei der Bearbeitung durch das Slow-Scannersystem 3 entlang der Bearbeitungslinie s_{y} in y-Richtung die Auslenkungsamplitude A des Fast-Scannersystems 5 auf die definierte Scannbreite E und die Auslenkungsamplitude B auf Null gesetzt wird. Im Übergangsbereich U von der Bearbeitung in x-Richtung auf die Bearbeitung in y-Richtung werden die Auslenkungsamplituden A, B des Fast-Scannersystems 5 beispielsweise kontinuierlich erhöht respektive reduziert.

In den Figuren 5-7 stellen die Kreise jeweils schematisch die Fokusmittelpunkte eines gepulsten Laserstrahls (Bearbeitungsstrahl L5) respektive eines Femtosekundenlaserpulses im Fokus Q dar. Die in den Figuren 5, 6, 7, 8a und 8b dargestellten Scanntrajektorien t weisen in bestimmten Bereichen ungleichmässig verteilte Laserpulse auf. Als Massnahme dagegen werden je nach Ausführungsvariante und/oder Anwendung der Laserstrahl respektive die Laserpulse durch das Filtermodul 4 ausgeblendet, beispielsweise mittels eines Shutters oder einer Blende 41, wodurch selektiv bestimmte Bereiche der Scanntrajektorie t ausgewählt werden. Im Beispiel der Figur 5 werden beispielsweise optional die Schwingungsspitzen, welche vergleichsweise dichter aufeinanderfolgende Laserpulse aufweisen, in den Bereichen R1 durch das Filtermodul 4 ausgeblendet, vorzugsweise durch eine Feldblende. Im Beispiel der Figur 6, werden jeweils die aufsteigenden Flanken der Scanntrajekorie t im Bereich R2 ausgeblendet, so dass selektiv nur die absteigenden Äste der Scanntrajektorie t zur Bearbeitung verwendet werden, wobei beispielsweise auch die Schwingungsspitzen abgeschnitten werden. Vorzugsweise wird der Bereich R2 durch einen Shutter ausgeblendet der beim Laser angeordnet und der Strahlquelle 6 nachgeschaltet ist und zum Ausblenden des Bereichs R2 geschlossen und andernfalls geöffnet wird. Um den Pulsabstand innerhalb eines Astes respektive einer Flanke anzugleichen, wird in einer weiteren Ausführungsvariante durch das Steuermodul 7 die Pulsfrequenz abhängig von der Position in der Scanntrajektorie t angepasst werden (z.B. über Pulspicker) oder die Auslenkungsamplitude A, B des Fast-Scannersystems 5 dynamisch verändert. Durch die resultierende dynamische Vergrösserung (Streckung) respektive Verkleinerung (Stauchung) der Auslenkungsamplitude F der Scannbewegung f kann der Abstand zwischen nacheinander folgenden einzelnen Femtosekundenlaserpulsen auf der Scannkurve f respektive Scanntrajektorie t einfach und unabhängig von s vergrössert respektive verkleinert werden und dadurch die Anzahl Laserpulse innerhalb der nicht ausgeblendeten Scannbreite E variabel gesteuert werden.

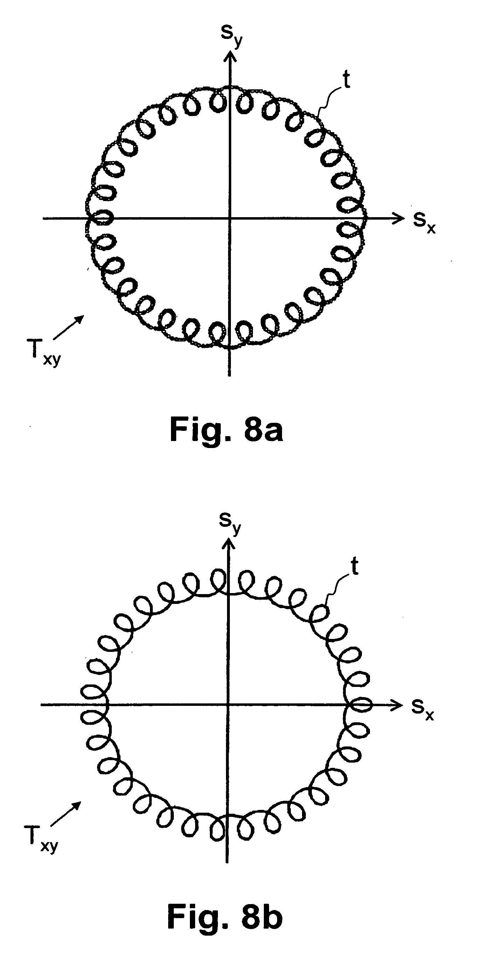

Figuren 8a und 8b zeigen Beispiele von zykloidförmigen Scanntrajektorien t, die durch Überlagerung von runden Scannkurven f des Fast-Scannersystems 5 auf runde Bearbeitungslinien s des Slow-Scannersystems 3 erzeugt werden. Wie in den Figuren 8a und 8b schematisch dargestellt ist, ist das Steuermodul 7 respektive die Fastscannersteuerung eingerichtet, das Fast-Scannersystem 5 derart zu steuern, dass die Laserpulse auf der Projektionsebene P_{xy} auf eine runde, beispielsweise kreisförmige oder elliptische, Scannkurve f abgelenkt werden, und das Steuermodul 7 respektive die Slowscannersteuerung ist eingerichtet, das Slow-Scannersystem 3 derart zu steuern, dass die vom Fast-Scannersystem 5 abgelenkten Femtosekundenlaserpulse L2 respektive die vom Filtermodul 4 nicht ausgeblendeten Femtosekundenlaserpulse L3 auf eine runde Bearbeitungslinie s abgelenkt respektive überlagert werden, beispielsweise eine kreisförmige oder elliptische Bearbeitungslinie s. Wie aus den Figuren 8a und 8b ersichtlich ist unterscheiden sich die resultierenden zykloidförmigen Scanntrajektorien t dadurch, dass die überlagerten Scannkurven f einen umgekehrten Drehsinn aufweisen.

Eine besonders optimale Ausgestaltung einer zykloidförmigen Scanntrajektorie t ergibt sich aus der Grösse der Wechselwirkungszonen, welche grösser als der Spotdurchmesser eines Laserpulses sein können (Kavitationsblasen). Je nach Anwendung ist es durchaus sinnvoll, wenn sich Scanntrajektorien t überlagern, beispielsweise können die Schlaufen der Zykloiden in die Lücken benachbarter Zykloide fallen. Diese letztere Variante hat den Vorteil, dass sie steuerungstechnisch einfach ist, weil sie mit geringer oder ohne Kopplung zum Slow-Scannersystem 3 ausführbar ist.

Abschliessend soll angeführt werden, dass in der Beschreibung zwar Computerprogrammcode spezifischen funktionalen Modulen zugeordnet wurde, dass der Fachmann jedoch verstehen wird, dass der Computerprogrammcode unterschiedlich strukturiert geändert werden kann, ohne dabei vom Schutzgegenstand abzuweichen.

## Patentansprüche

1. Ophthalmologische Vorrichtung (1) zum Bearbeiten von Augengewebe (8) mittels Femtosekundenlaserpulsen, umfassend:
eine Projektionsoptik (2) eingerichtet zur fokussierten Projektion der Femtosekundenlaserpulse ins Augengewebe (8),
ein erstes der Projektionsoptik (2) vorgelagertes strahlablenkendes Scannersystem (3) eingerichtet zum Scannen des Augengewebes (8) mit den Femtosekundenlaserpulsen entlang einer Bearbeitungslinie (s),
ein zweites dem ersten Scannersystem (3) vorgelagertes strahlablenkendes Scannersystem (5) eingerichtet zum Scannen des Augengewebes (8) mit den Femtosekundenlaserpulsen auf einer der Bearbeitungslinie (s) überlagerten Scannkurve (f), mit einer Scanngeschwindigkeit, die ein Vielfaches der Scanngeschwindigkeit des ersten Scannersystems (3) beträgt, umfassend mindestens einen Ablenkungsspiegel und eine erste Scannachse (51) und eine zweite Scannachse (52), und
ein Steuermodul (7) eingerichtet zum Steuern der Ablenkung (A, b) der Femtosekundenlaserpulse um die erste Scannachse (51) und der Ablenkung der Femtosekundenlaserpulse um die zweite Scannachse (52) gemäss mindestens einem aus: definierte Kurvenform, definierte Kurvenamplitude und definierte Kurvenausrichtung der Scannkurve (f), **dadurch gekennzeichnet, dass** das Steuermodul (7) eingerichtet ist, abhängig von der Richtung der Bearbeitungslinie (s) die Auslenkungsamplitude (B) der Ablenkung (b) um die erste Scannachse (51) und die Auslenkungsamplitude (A) der Ablenkung (a) um die zweite Scannachse (52) zu steuern.

2. Vorrichtung (1) nach Anspruch 1, **dadurch gekennzeichnet, dass** das Steuermodul (7) eingerichtet ist, die Kurvenausrichtung der Scannkurve (f) durch gekoppelte Steuerung von Auslenkungsamplitude (B) der Ablenkung (b) um die erste Scannachse (51) und von Auslenkungsamplitude (A) der Ablenkung (a) um die zweite Scannachse (52) zu bestimmen.

3. Vorrichtung (1) nach Anspruch 1, **dadurch gekennzeichnet, dass** das Steuermodul (7) eingerichtet ist, die Kurvenausrichtung der Scannkurve (f) durch gekoppelte Steuerung von Auslenkungsamplitude (B) der Ablenkung (b) um die erste Scannachse (51), von Auslenkungsamplitude (A) der Ablenkung (a) um die zweite Scannachse (52), und von der relativen Phase von der Ablenkung (b) um die erste Scannachse (51) und der Ablenkung (a) um die zweite Scannachse (52) zu bestimmen.

4. Vorrichtung (1) nach einem der Ansprüche 1 bis 3, **dadurch gekennzeichnet, dass** das Steuermodul (7) eingerichtet ist, die Kurvenamplitude (F) der Scannkurve (f) durch gekoppelte Steuerung von Auslenkungsamplitude (B) der Ablenkung (b) um die erste Scannachse (51) und von Auslenkungsamplitude (A) der Ablenkung (a) um die zweite Scannachse (52) zu bestimmen.

5. Vorrichtung (1) nach einem der Ansprüche 1 bis 4, **dadurch gekennzeichnet, dass** das Steuermodul (7) eingerichtet ist, die Kurvenform der Scannkurve (f) durch Steuerung der relativen Phase von der Ablenkung (b) um die erste Scannachse (51) und der Ablenkung (a) um die zweite Scannachse (52) zu bestimmen.

6. Vorrichtung (1) nach einem der Ansprüche 1 bis 5, **dadurch gekennzeichnet, dass** das Steuermodul (7) eingerichtet ist, die Kurvenform der Scannkurve (f) durch gekoppelte Steuerung von Auslenkungsamplitude (B) der Ablenkung (b) um die erste Scannachse (51) und von Auslenkungsamplitude (A) der Ablenkung (a) um die zweite Scannachse (52) zu bestimmen.

7. Vorrichtung (1) nach einem der Ansprüche 1 bis 6, **dadurch gekennzeichnet, dass** das Steuermodul (7) eingerichtet ist, die Kurvenform der Scannkurve (f) durch gekoppelte Steuerung der Ablenkungsfrequenz (ωₓ) um die erste Scannachse (51) und der Ablenkungsfrequenz (w_{y}) um die zweite Scannachse (52) zu bestimmen.

8. Vorrichtung (1) nach einem der Ansprüche 5 bis 7, **dadurch gekennzeichnet, dass** das Steuermodul (7) eingerichtet ist, eine durch Fouriersynthese definierbare Kurvenform der Scannkurve (f) durch Steuerung von mindestens einem zu bestimmen aus: Auslenkungsamplitude (B) der Ablenkung (b) um die erste Scannachse (51), Auslenkungsamplitude (A) der Ablenkung (a) um die zweite Scannachse (52), relative Phase von der Ablenkung (b) um die erste Scannachse (51) und der Ablenkung (a) um die zweite Scannachse (52), Ablenkungsfrequenz (ωₓ) um die erste Scannachse (51), und Ablenkungsfrequenz (ω_{y}) um die zweite Scannachse (52).

9. Vorrichtung (1) nach einem der Ansprüche 1 bis 8, **dadurch gekennzeichnet, dass** das Steuermodul (7) eingerichtet ist, abhängig von der Bearbeitungslinie (s) mindestens eines zu steuern aus: Ablenkungsfrequenz (ωₓ) um die erste Scannachse (51) und Ablenkungsfrequenz (ω_{y}) um die zweite Scannachse (52).

10. Vorrichtung (1) nach einem der Ansprüche 1 bis 9, **dadurch gekennzeichnet, dass** das Steuermodul (7) eingerichtet ist, bei einer Richtungsänderung der Bearbeitungslinie (sₓ, s_{y}) die Auslenkungsamplitude (B) der Ablenkung (b) um die erste Scannachse (51) zu reduzieren und die Auslenkungsamplitude (A) der Ablenkung (a) um die zweite Scannachse (52) zu erhöhen.

11. Vorrichtung (1) nach einem der Ansprüche 1 bis 10, **dadurch gekennzeichnet, dass** das Steuermodul (7) eingerichtet ist, die Ablenkung (b) um die erste Scannachse (51) und die Ablenkung (a) um die zweite Scannachse (52) so zu steuern, dass die Ablenkung (b) um die zweite Scannachse (52) mit einer doppelt so hohen Ablenkungsfrequenz (ω_{y}) wie die Ablenkung (b) um die erste Scannachse (51) erfolgt und die Ablenkung (a) um die zweite Scannachse (52) eine mindestens teilweise Kompensation der durch das erste Scannersystem (3) bewirkten Ablenkung in Richtung der Bearbeitungslinie (sₓ) bewirkt.

12. Vorrichtung (1) nach einem der Ansprüche 1 bis 11, **gekennzeichnet durch** eine zwischen dem ersten Scannersystem (3) und dem zweiten Scannersystem (5) angeordnete Blende (41) zum Ausblenden von Femtosekundenlaserpulsen, die vom zweiten Scannersystem (5) in einen Bereich (R1) ausserhalb einer definierten Scannbreite abgelenkt werden.

13. Vorrichtung (1) nach einem der Ansprüche 1 bis 12, **gekennzeichnet durch** ein steuerbares Filtermodul (4) zum selektiven Ausschliessen von Femtosekundenlaserpulsen in einem definierten Bereich (R1, R2) der Scannkurve (f).

14. Vorrichtung (1) nach einem der Ansprüche 1 bis 13, **dadurch gekennzeichnet, dass** die erste Scannachse (51) und die zweite Scannachse (52) des zweiten Scannersystems (5) orthogonal zueinander ausgerichtet sind, dass das zweite Scannersystem (5) eingerichtet ist, die Femtosekundenlaserpulse oszillierend um die erste Scannachse (51) und um die zweite Scannachse (52) abzulenken, und dass das erste Scannersystem (3) einen im Vergleich zum zweiten Scannersystem (5) wesentlich grösseren Auslenkungsgrad aufweist.

15. Vorrichtung (1) nach einem der Ansprüche 1 bis 14, **dadurch gekennzeichnet, dass** das Steuermodul (7) eingerichtet ist, die Ablenkung der Femtosekundenlaserpulse um die erste Scannachse (51) und die Ablenkung der Femtosekundenlaserpulse um die zweite Scannachse (52) so zu steuern, dass die Scannkurve (f) eine runde Kurvenform aufweist, und dass das Steuermodul (7) eingerichtet ist, die Scanngeschwindigkeit des ersten Scannersystems (3) so zu steuern, dass sich aus der Überlagerung von der Scannkurve (f) auf die Bearbeitungslinie (s) eine resultierende Scanntrajektorie (t) mit einem definierten Trajektorienverlauf ergibt.

## Claims

1. Ophthalmological device (1) for processing eye tissue (8) by means of femtosecond laser pulses comprising:
a projection optical unit (2) designed for the focused projection of the femtosecond laser pulses into the eye tissue (8),
a first beam-deffecting scanner system (3), disposed upstream of the projection optical unit (2), designed for scanning the eye tissue (8) with the femtosecond laser pulses along a processing line (s),
a second beam-deflecting scanner system (5) disposed upstream of the first scanner system (3), designed for scanning the eye tissue (8) with the femtosecond laser pulses on a scanning curve (f) superimposed on the processing line (s), with a scanning speed that is a multiple of the scanning speed of the first scanner system (3), comprising at least one deflection mirror and a first scanning axis (51) and a second scanning axis (52), and
a control module (7) designed for controlling the deflection (A), (b) of the femtosecond laser pulses about the first scanning axis (51) and the deflection of the femtosecond laser pulses about the second scanning axis (52) in accordance with at least one from: defined curve shape, defined curve amplitude and defined curve orientation of the scanning curve (f), **characterized in that** the control module (7) is designed to control, depending on the direction of the processing line(s), the excursion amplitude (B) of the deflection (b) about the first scanning axis (51), and the excursion amplitude (A) of the deflection (a) about the second scanning axis (52).

2. Device (1) according to Claim 1, **characterized in that** the control module (7) is designed to determine the curve orientation of the scanning curve (f) by coupled control of excursion amplitude (B) of the deflection (b) about the first scanning axis (51) and of excursion amplitude (A) of the deflection (a) about the second scanning axis (52).

3. Device (1) according to Claim 1, **characterized in that** the control module (7) is designed to determine the curve orientation of the scanning curve (f) by coupled control of excursion amplitude (B) of the deflection (b) about the first scanning axis (51), of excursion amplitude (A) of the deflection (a) about the second scanning axis (52), and of the relative phase of the deflection (b) about the first scanning axis (51) and of the deflection (a) about the second scanning axis (52).

4. Device (1) according to any of Claims 1 to 3, **characterized in that** the control module (7) is designed to determine the curve amplitude (F) of the scanning curve (f) by coupled control of excursion amplitude (B) of the deflection (b) about the first scanning axis (51) and of excursion amplitude (A) of the deflection (a) about the second scanning axis (52).

5. Device (1) according to any of Claims 1 to 4, **characterized in that** the control module (7) is designed to determine the curve shape of the scanning curve (f) by control of the relative phase of the deflection (b) about the first scanning axis (51) and of the deflection (a) about the second scanning axis (52).

6. Device (1) according to any of Claims 1 to 5, **characterized in that** the control module (7) is designed to determine the curve shape of the scanning curve (f) by coupled control of the excursion amplitude (B) of the deflection (b) about the first scanning axis (51) and of the excursion amplitude (A) of the deflection (a) about the second scanning axis (52).

7. Device (1) according to any of Claims 1 to 6, **characterized in that** the control module (7) is designed to determine the curve shape of the scanning curve (f) by coupled control of the deflection frequency (ωₓ) about the first scanning axis (51) and of the deflection frequency (ω_{y}) about the second scanning axis (52).

8. Device (1) according to any of Claims 5 to 7, **characterized in that** the control module (7) is designed to determine a curve shape - definable by Fourier synthesis - of the scanning curve (f) by control of at least one from: excursion amplitude (B) of the deflection (b) about the first scanning axis (51), excursion amplitude (A) of the deflection (a) about the second scanning axis (52), relative phase of the deflection (b) about the first scanning axis (51) and of the deflection (a) about the second scanning axis (52), deflection frequency (ωₓ) about the first scanning axis (51), and deflection frequency (ω_{y}) about the second scanning axis (52).

9. Device (1) according to any of Claims 1 to 8, **characterized in that** the control module (7) is designed to control, depending on the processing line (s) at least one from: deflection frequency (ωₓ) about the first scanning axis (51), and deflection frequency (ω_{y}) about the second scanning axis (52).

10. Device (1) according to any of Claims 1 to 9, **characterized in that** the control module (7) is designed, in the case of a change in the direction of the processing line (sₓ, s_{y}), to reduce the excursion amplitude (B) of the deflection (b) about the first scanning axis (51) and to increase the excursion amplitude (A) of the deflection (a) about the second scanning axis (52).

11. Device (1) according to any of Claims 1 to 10, **characterized in that** the control module (7) is designed to control the deflection (b) about the first scanning axis (51) and the deflection (a) about the second scanning axis (52) such that the deflection (b) about the second scanning axis (52) is effected with a deflection frequency (ω_{y}) having a doubled magnitude relative to the deflection (b) about the first scanning axis (51), and the deflection (a) about the second scanning axis (52) brings about at least partial compensation of the deflection brought about by the first scanner system (3) in the direction of the processing line (sₓ).

12. Device (1) according to any of Claims 1 to 11, **characterized by** a diaphragm (41) arranged between the first scanner system (3) and the second scanner system (5) and serving for masking out femtosecond laser pulses that are deflected by the second scanner system (5) into a region (R1) outside a defined scanning width.

13. Device (1) according to any of Claims 1 to 12, **characterized by** a controllable filter module (4) for selectively excluding femtosecond laser pulses in a defined region (R1, R2) of the scanning curve (f).

14. Device (1) according to any of Claims 1 to 13, **characterized in that** the first scanning axis (51) and the second scanning axis (52) of the second scanner system (5) are oriented orthogonally with respect to one another, **in that** the second scanner system (5) is designed to deflect the femtoscond laser pulses in an oscillating fashion about the first scanning axis (51) and about the second scanning axis (52), and **in that** the first scanner system (3) has a significantly greater degree of excursion in comparison with the second scanner system (5).

15. Device (1) according to any of Claims 1 to 14, **characterized in that** the control module (7) is designed to control the deflection of the femtosecond laser pulses about the first scanning axis (51) and the deflection of the femtosecond laser pulses about the second scanning axis (52) such that the scanning curve (f) has a round curve shape, and **in that** the control module (7) is designed to control the scanning speed of the first scanner system (3) such that a resulting scanning trajectory (t) having a defined trajectory profile arises from the superimposition of the scanning curve (f) onto the processing line (s).

## Revendications

1. Dispositif ophtalmologique (1) destiné au traitement de tissus oculaires (8) au moyen d'impulsions laser femtoseconde, comprenant :
une optique de projection (2) conçue pour projeter de manière focalisée les impulsions laser femtoseconde dans le tissu oculaire (8),
un premier système de balayage (3) de déviation de faisceaux disposé en amont de l'optique de projection (2), conçu pour balayer le tissu oculaire (8) avec les impulsions laser femtoseconde le long d'une ligne de traitement (s),
un second système de balayage (5) de déviation de faisceaux disposé en amont du premier système de balayage (3), conçu pour balayer le tissu oculaire (8) avec les impulsions laser femtoseconde sur une courbe de balayage (f) superposé à une ligne de traitement (s), à une vitesse de balayage égale à un multiple de la vitesse de balayage du premier système de balayage (3), comprenant au moins un miroir de déviation et un premier axe de balayage (51) et un second axe de balayage (52), et
un module de commande (7) conçu pour commander la déviation (A, b) des impulsions laser femtoseconde autour du premier axe de balayage (51) et la déviation des impulsions laser femtoseconde autour du second axe de balayage (52) conformément à au moins l'un des éléments suivants : une forme de courbe définie, une amplitude de courbe définie et une direction de courbe définie de la courbe de balayage (f), **caractérisé en ce que** le module de commande (7) est conçu pour commander, en fonction de la direction de la ligne de traitement (s), l'amplitude de déviation (B) de la déviation (b) autour du premier axe de balayage (51) et l'amplitude de déviation (A) de la déviation (a) autour du second axe de balayage (52).

2. Dispositif (1) selon la revendication 1, **caractérisé en ce que** le module de commande (7) est conçu pour déterminer la direction de courbe de la courbe de balayage (f) au moyen d'une commande couplée de l'amplitude de déviation (B) de la déviation (b) autour du premier axe de balayage (51) et de l'amplitude de déviation (A) de la déviation (a) autour du second axe de balayage (52).

3. Dispositif (1) selon la revendication 1, **caractérisé en ce que** le module de commande (7) est conçu pour déterminer la direction de courbe de la courbe de balayage (f) au moyen d'une commande couplée de l'amplitude de déviation (B) de la déviation (b) autour du premier axe de balayage (51), de l'amplitude de déviation (A) de la déviation (a) autour du second axe (52), et de la phase relative de la déviation (b) autour du premier axe de balayage (51) et de la déviation (a) autour du second axe de balayage (52).

4. Dispositif (1) selon l'une quelconque des revendications 1 à 3, **caractérisé en ce que** le module de commande (7) est conçu pour déterminer l'amplitude de courbe (F) de la courbe de balayage (f) au moyen d'une commande couplée de l'amplitude de déviation (B) de la déviation (b) autour du premier axe de balayage (51) et de l'amplitude de déviation (A) de la déviation (a) autour du second axe de balayage (52).

5. Dispositif (1) selon l'une quelconque des revendications 1 à 4, **caractérisé en ce que** le module de commande (7) est conçu pour déterminer la forme de courbe de la courbe de balayage (f) au moyen d'une commande de la phase relative de la déviation (b) autour du premier axe de balayage (51) et de la déviation (a) autour du second axe de balayage (52).

6. Dispositif (1) selon l'une quelconque des revendications 1 à 5, **caractérisé en ce que** le module de commande (7) est conçu pour déterminer la forme de courbe de la courbe de balayage (f) au moyen d'une commande couplée de l'amplitude de déviation (B) de la déviation (b) autour du premier axe de balayage (51) et de l'amplitude de déviation (A) de la déviation (a) autour du second axe de balayage (52).

7. Dispositif (1) selon l'une quelconque des revendications 1 à 6, **caractérisé en ce que** le module de commande (7) est conçu pour déterminer la forme de courbe de la courbe de balayage (f) au moyen d'une commande couplée de la fréquence de déviation (ωₓ) autour du premier axe de balayage (51) et de la fréquence de déviation (ω_{y}) autour du second axe de balayage (52).

8. Dispositif (1) selon l'une quelconque des revendications 5 à 7, **caractérisé en ce que** le module de commande (7) est conçu pour déterminer une forme de courbe de la courbe de balayage (f) définissable par synthèse de Fourier au moyen d'une commande d'au moins l'un des éléments suivants : l'amplitude de déviation (B) de la déviation (b) autour du premier axe de balayage (51), l'amplitude de déviation (A) de la déviation (a) autour du second axe de balayage (52), la phase relative de la déviation (b) autour du premier axe de balayage (51) et la déviation (a) autour du second axe de balayage (52), la fréquence de déviation (ωₓ) autour du premier axe de balayage (51) et la fréquence de déviation (ω_{y}) autour du second axe de balayage (52).

9. Dispositif (1) selon l'une quelconque des revendications 1 à 8, **caractérisé en ce que** le module de commande (7) est conçu pour commander, en fonction de la ligne de traitement (s), au moins l'un des éléments suivants : la fréquence de déviation (ωₓ) autour du premier axe de balayage (51) et la fréquence de déviation (ω_{y}) autour du second axe de balayage (52).

10. Dispositif (1) selon l'une quelconque des revendications 1 à 9, **caractérisé en ce que** le module de commande (7) est conçu pour réduire l'amplitude de déviation (B) de la déviation (b) autour du premier axe de balayage (51) et pour augmenter l'amplitude de déviation (A) de la déviation (a) autour du second axe de balayage (52) lors d'une modification de direction de la ligne de traitement (sₓ, s_{y}).

11. Dispositif (1) selon l'une quelconque des revendications 1 à 10, **caractérisé en ce que** le module de commande (7) est conçu pour commander la déviation (b) autour du premier axe de balayage (51) et la déviation (a) autour du second axe de balayage (52) de manière à ce que la déviation (b) autour du second axe de balayage (52) s'effectue avec une fréquence de déviation (ω_{y}) deux fois plus élevée que la déviation (b) autour du premier axe de balayage (51) et à ce que la déviation (a) autour du second axe de balayage (52) produise une compensation au moins partielle de la déviation provoquée par le premier système de balayage (3) dans la direction de la ligne de traitement (sₓ).

12. Dispositif (1) selon l'une quelconque des revendications 1 à 11, **caractérisé par** un diaphragme (41) disposé entre le premier système de balayage (3) et le second système de balayage (5), destiné à occulter des impulsions laser femtoseconde qui sont déviées par le second système de balayage (5) dans une région (R1) située en dehors d'une largeur de balayage définie.

13. Dispositif (1) selon l'une quelconque des revendications 1 à 12, **caractérisé par** un module de filtrage (4) pouvant être commandé, destiné à exclure sélectivement des impulsions laser femtoseconde dans une région définie (R1, R2) de la courbe de balayage (f).

14. Dispositif (1) selon l'une quelconque des revendications 1 à 13, **caractérisé en ce que** le premier axe de balayage (51) et le second axe de balayage (52) du second système de balayage (5) sont orientés perpendiculairement d'un à l'autre, **en ce que** le second système de balayage (5) est conçu pour dévier de manière oscillante les impulsions laser femtoseconde autour du premier axe de balayage (51) et autour du second axe de balayage (52), et **en ce que** le premier système de balayage (3) présente une amplitude de déviation sensiblement plus élevée par comparaison au second système de balayage (5).

15. Dispositif (1) selon l'une quelconque des revendications 1 à 14, **caractérisé en ce que** le module de commande (7) est conçu pour commander la déviation des impulsions laser femtoseconde autour du premier axe de balayage (51) et la déviation des impulsions laser femtoseconde autour du second axe de balayage (52) de manière à ce que la courbe de balayage (f) présente une forme de courbe arrondie et **en ce que** le module de commande (7) est conçu pour commander la vitesse de balayage du premier système de balayage (3) de manière à ce qu'il se produise une trajectoire de balayage (t) résultante présentant une évolution de trajectoire définie à partir de la superposition de la courbe de balayage (f) à la ligne de traitement (s).
